(19) Europäisches Patentamt
European Patent Office
Office européen des brevets

(11) Numéro de publication: **0 206 863**
**A1**

(12) **DEMANDE DE BREVET EUROPEEN**

(21) Numéro de dépôt: **86401110.1**

(22) Date de dépôt: **27.05.86**

(51) Int. Cl.[4]: **C12N 15/00** , C12P 21/02 , C12N 1/20 , //(C12N1/20,C12R1:19)

Le demandeur a présenté une déclaration conformément à la règle 28 (4) CBE (remise d'un échantillon uniquement à un expert).

(30) Priorité: **28.05.85 FR 8507977**

(43) Date de publication de la demande: **30.12.86 Bulletin 86/52**

(84) Etats contractants désignés: **AT BE CH DE FR GB IT LI LU NL SE**

(71) Demandeur: **SANOFI, société anonyme**
**40, Avenue George V**
**F-75008 Paris(FR)**

(72) Inventeur: **Roskam, Willem**

**F-31450 - Montgiscard Majouret(FR)**
Inventeur: **Ferrara, Pascual**
**135, rue de la Salade Ponsan**
**F-31400 - Rangueil(FR)**

(74) Mandataire: **Gillard, Marie-Louise et al**
**Cabinet Beau de Loménie 55, Rue d'Amsterdam**
**F-75008 Paris(FR)**

(54) **Dérivés non amidés de la somatocrinine et procédé de préparation par génie génétique.**

(57) La présente invention concerne un procédé d'obtention de dérivés non amidés de la somatocrinine de formule GRF(1-43)-X, dans laquelle X est la leucine, la glycine, la valine ou l'alanine, par un procédé de génie génétique qui permet l'obtention desdits dérivés liés à une protéine porteuse par l'intermédiaire d'un résidu tryptophane et par coupure ultérieure du produit obtenu au niveau du tryptophane pour fournir lesdits dérivés.

EP 0 206 863 A1

# Dérivés non amidés de la somatocrinine et procédé de préparation par génie génétique.

La présente invention concerne des dérivés non amidés de la somatocrinine ainsi qu'un procédé de préparation desdits dérivés par génie génétique.

Dans la présente description, on désigne par "somatocrinine" ou "GRF" aussi bien la somatocrinine humaine que les somatocrinines animales, et par "GRF(1-43)-X" leurs dérivés non amidés, X étant un acide aminé choisi parmi les acides aminés : leucine, glycine, alanine et valine. Parmi ces dérivés, celui dans lequel X est la leucine est aussi dénommé GRF-OH.

La somatocrinine humaine, ou hGRF, est un peptide constitué par l'enchaînement de 44 aminoacides. Sa séquence est la suivante :

```
1               5                   10
H-Tyr-Ala-Asp-Ala-Ile-Phe-Thr-Asn-Ser-Tyr-Arg

            15                  20
-Lys-Val-Leu-Gly-Gln-Leu-Ser-Ala-Arg-Lys-Leu-Leu-

    25                  30                  35
Gln-Asp-Ile-Met-Ser-Arg-Gln-Gln-Gly-Glu-Ser-Asn-

                40              44
Gln-Glu-Arg-Gly-Ala-Arg-Ala-Arg-Leu-NH2
```

Il a été récemment découvert par A. Guillemin et coll. (Science, 218 585-587, 1982) à partir d'extraits d'une tumeur pancréatique humaine.

Le GRF est particulièrement actif sur la stimulation de la libération de l'hormone de croissance - (GH) aussi bien in vitro qu'in vivo. In vitro, en particulier, son efficacité se manifeste à des doses de quelques femto moles/ml (DE50 = 15 femto moles/ml). L'intérêt thérapeutique en médecine humaine de cette substance va donc se situer au niveau du traitement du nanisme et des retards à la croissance en pédiatrie. D'autres applications sont possibles dans les cas de déficience anabolique protéique (ulcères de stress, réparation de fractures ou d'atteintes du cartilage, brûlures étendues -pendant la phase anabolique -, réparations cutanées, ostéoporoses).

Dans le domaine vétérinaire, ce composé présente un intérêt évident au niveau de la croissance pondérale des animaux d'élevage (bovins, ovins...).

Le développement industriel du GRF nécessite la synthèse de quantités importantes de celui-ci. Les procédés classiques de synthèse en phase solide permettent l'obtention en des temps courts de faibles quantités de ce peptide (Science, 218, 585-587, 1982) à des coûts très élevés et incompatibles avec un développement pharmaceutique à grande échelle.

On sait que les polypeptides peuvent être préparés par des techniques de génie génétique en incorporant un ADN recombinant codant pour lesdits polypeptides dans une cellule hôte et en faisant exprimer ledit ADN recombinant à l'intérieur de cette cellule hôte.

Cette méthode directe ne peut cependant pas être appliquée dans l'expression du gène codant pour le GRF, notamment dans E. coli, parce que le GRF est très sensible à l'action protéolytique intracellulaire.

Dans le cas de la somatostatine, on a protégé cette hormone en la faisant exprimer avec une protéine porteuse, le gène de la somatostatine étant séparé du gène codant pour la protéine porteuse par un codon ATG (codant pour la méthionine) situé à l'extrémité du gène codant pour la somatostatine correspondant à la partie N terminale de celle-ci (Itakura et al., Science 1977, 198, 1056-1063). La protéine hybride obtenue après l'expression peut être coupée au niveau de la méthionine, à l'aide du bromure de cyanogène, pour libérer la somatostatine.

Ce procédé ne peut pas être appliqué au GRF parce que le bromure de cyanogène provoque aussi la coupure de la molécule du produit souhaité.

Des coupures au niveau d'autres acides aminés dans des polypeptides ont été décrites, mais de telles méthodes n'ont pas la spécificité nécessaire pour l'obtention d'un produit de pureté satisfaisante avec un rendement satisfaisant.

On a maintenant trouvé que les dérivés GRF(1-43)-X dans lesquels X est tel que défini précédemment peuvent être préparés à l'échelle industrielle par un procédé de génie génétique qui permet l'obtention desdits dérivés liés à une protéine porteuse par l'intermédiaire d'un résidu tryptophane. La protéine hybride résultante est ensuite coupée au niveau du tryptophane pour fournir lesdits dérivés.

Cette coupure est réalisée sélectivement au niveau du tryptophane par action d'un agent oxydant, tel que la 2-(2-nitrophénylsulfényl)-3'-bromo-3-méthylindolénine, ci-après désigné BNPS-scatole.

Ainsi, selon un de ses aspects la présente invention concerne un procédé pour l'obtention du GRF-OH par génie génétique qui consiste à :

1) préparer le fragment d'ADN codant pour le Trp-GRF(1-43)-X;

2) insérer ledit fragment dans un vecteur contenant le gène codant pour la protéine porteuse avec les éléments génétiques nécessaires à son expression, l'insertion étant réalisée au niveau de la séquence codant pour la partie carboxylique terminale de ladite protéine porteuse;

3) transformer une cellule hôte avec le vecteur recombinant résultant;

4) faire exprimer le polypeptide de formule I :

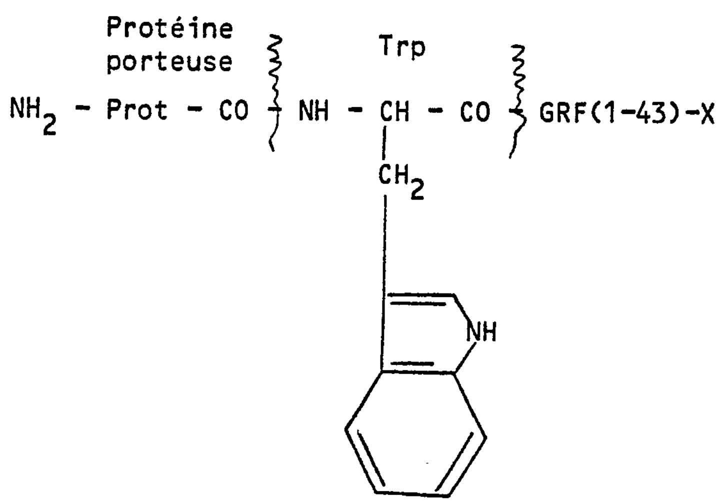

(I)

X = Leu, Ala, Val ou Gly.

5) récupérer ledit polypeptide de formule I et le couper au niveau du tryptophane à l'aide d'un agent oxydant pour obtenir le composé de formule GRF(1-43)-X dans laquelle X est tel que défini ci-dessus.

Le fragment d'ADN codant pour le Trp-GRF(1-43)-X mis en oeuvre dans le procédé selon l'invention peut être obtenu par le procédé qui consiste à :

-synthétiser chimiquement un oligonucléotide portant le codon du tryptophane;

-unir ledit oligonucléotide au gène codant pour le GRF(1-43)-X porté par un vecteur à l'extrémité correspondant à la partie N terminale de celui-ci et

-isoler ledit fragment d'ADN résultant par des méthodes classiques.

L'oligonucléotide synthétisé est choisi pour permettre la fusion en phase de lecture du gène codant pour le Trp-GRF(1-43)-X avec celui de la protéine porteuse.

De préférence, le gène codant pour le GRF(1-43)-X dans lequel X = leucine ou GRF-OH est obtenu à partir du plasmide pUC9 (Messing and Vieira, Gene, 19 (1982) 259) disponible sur le marché, qui contient le gène codant pour la méthionine-GRF-OH de formule :

$$CH_3-S-CH_2-CH_2-\underset{NH_2}{\underset{|}{CH}}-CO-GRF-OH \qquad (II)$$

La deuxième étape du procédé de l'invention consiste à insérer le fragment d'ADN obtenu dans un vecteur contenant le gène codant pour la protéine porteuse avec les éléments génétiques nécessaires à son expression. La protéine porteuse est une protéine qui est exprimée efficacement par la cellule hôte. Cette protéine porteuse est de préférence la $\beta$-galactosidase lorsque la cellule hôte est E. coli.

Selon un mode opératoire, le fragment d'ADN codant pour Trp-GRF(1-43)-X est inséré dans un vecteur contenant le gène de la protéine porteuse et les éléments génétiques nécessaires à l'expression.

Suivant un autre mode opératoire, on insère ledit fragment d'ADN dans un vecteur contenant le gène codant pour la protéine porteuse; on isole le gène résultant et on l'insère dans un nouveau vecteur contenant les éléments génétiques nécessaires à l'expression.

La cellule hôte utilisée dans le procédé de l'invention peut être une cellule procaryote ou eucaryote. Un procaryote préféré est l'espèce bactérienne Escherichia coli.

A titre d'exemples de cellules appropriées, on peut citer les cellules de E. coli dénommées CAG 1139 (GROSSMAN A.D. et al. Cell, 32 (1983) 151-159) et MC 1061 (CASADABAN M. et al. J. Bacteriol. (1980), 143, 971-980).

Selon un autre aspect, l'invention a pour objet le vecteur recombinant codant pour la protéine hybride constituée par le GRF(1-43)-X lié par son extrémité N terminale à une protéine porteuse par l'intermédiaire d'un tryptophane.

Un vecteur recombinant préféré inclut la séquence d'ADN codant pour la $\beta$-galactosidase, un analogue, ou un fragment de ceux-ci, lié du côté de son carboxyle terminal au GRF(1-43)-X par l'intermédiaire du tryptophane.

Le vecteur recombinant est mis sous contrôle quant à son expression (transcription et traduction) d'éléments génétiques appropriés selon les techniques de génie génétique habituelles. De préférence, l'expression du gène est conditionnelle, à savoir elle est amorcée par un inducteur chimique, par exemple l'IPTG (isopropylthio-$\beta$-D-galactoside), ou par des conditions physiques, par exemple la température.

Ensuite, le gène, avec ses éléments de régulation, est introduit dans un ADN vecteur permettant l'obtention et la sélection des cellules hôtes transformées, ledit ADN vecteur étant de préférence un plasmide.

Les cellules transformées sont isolées selon les techniques habituelles et cultivées dans des conditions qui permettent l'expression du gène afin d'obtenir la protéine hybride souhaitée.

L'invention concerne également les souches de cellules hôtes contenant le vecteur recombinant défini ci-dessus. A titre d'exemple de telles souches, on citera la souche de E. coli MC 1061 contenant le vecteur recombinant préféré (plasmide 202,17) déposée à la "Collection Nationale de Cultures de Micro-organismes" à Paris, le 22 octobre 1984 sous le n° I-354.

Selon un autre de ses aspects, la présente invention a pour objet la protéine hybride de formule :

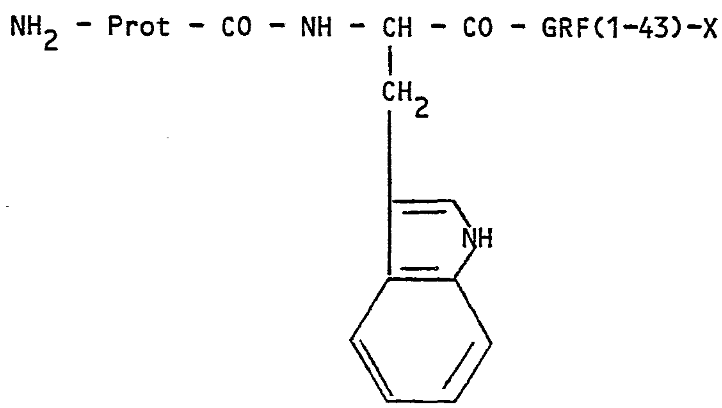

(I)

dans laquelle X représente un des acides aminés cités ci-dessus et Prot représente le résidu du côté de l'acide carboxylique terminal d'une protéine porteuse.

Une protéine hybride préférée est un composé de formule I ci-dessus dans laquelle Prot représente un résidu du côté de l'acide carboxylique terminal de la $\beta$-galactosidase, un analogue, ou un fragment de cette molécule.

La protéine hybride est extraite du milieu de culture ou des cellules transformées et purifiée par exemple par chromatographie.

La protéine hybride de formule I ainsi obtenue est coupée par le BNPS-scatole selon les techniques habituelles (H.M. Steinmann, J. Biol. Chem. 1978, 251, 8708-8720) et le dérivé GRF(1-43)-X est purifié par extraction, gel filtration et HPLC selon les méthodes connues (R. Guillemin et al., Science 1982, 218, 185-187).

Selon un autre de ses aspects, la présente invention concerne le polypeptide de formule

GRF(1-43)-X (II)

dans laquelle X est un acide aminé choisi parmi la leucine, la valine, l'alanine ou la glycine, qui représente un intermédiaire clé pour l'obtention du GRF.

L'exemple suivant illustre l'invention sans toutefois la limiter. Les détails expérimentaux des techniques de génie génétique sont décrits dans Molecular Cloning par T. Maniatis, Ed. Cold Spring Harbor Labs. USA. 1982.

EXEMPLE 1

1.a. Préparation du gène codant pour le Trp-hGRF-OH ou dérivé Trp-hGRF(1-43)-Leu

Un oligonucléotide double brin est préparé en hybridant un premier oligonucléotide de séquence 5'-OH-ACCACAGCTGG phosphorylé à son extrémité 5' terminale, avec un second oligonucléotide de séquence 5'-OH-AATTC-CAGCTGTGGT.

Cet oligonucléotide est ensuite fusionné avec l'ADN double brin du plasmide pUC9 (CREATIVE BIOMOLECULES, San Francisco, Ca, 94080, USA) -contenant le gène codant pour le Met-GRF-OH - ouvert par action préalable de l'enzyme de restriction RsaI (figure 1). Ledit oligonucléotide vient se positionner au niveau du deuxième nucléotide du gène codant pour le GRF-OH et du nucléotide qui lui est complémentaire (figure 2). La fusion est réalisée en incubant ledit oligonucléotide (en excès molaire de 50 fois par rapport aux extrémités RsaI du plasmide) êt le plasmide pUC9 ouvert par l'enzyme, en présence de l'ADN-ligase du bactériophage T4.

Le nouvel ADN double brin ainsi obtenu contient le gène codant pour le Trp-GRF-OH. L'action de l'enzyme de restriction SalI permet précisément de disposer, aprés purification par électrophorèse sur gel, du fragment d'ADN contenant ce gène et représenté sur la figure 2. Ce fragment est ensuite cloné dans Escherichia coli (souche MC 1061) en utilisant comme vecteur le grand fragment EcoRI-SalI du plasmide pBR322.

1.b. Fusion du gène codant pour le Trp-hGRF-OH avec un gène codant pour un fragment modifié de la $\beta$-qalactosidase placé sous le contrôle du promoteur PR.

Le plasmide pDIA5501 (P. Leplatois et A. Danchin, Biochimie, 1983, 65, 317-324) est un plasmide dérivé du plasmide pBR 322 qui contient le gène codant pour la $\beta$-galactosidase. Ce plasmide pDIA5501 est modifié en restituant sur ledit gène un site EcoRI, par coupure par l'enzyme de restriction SacI, puis fusion avec un fragment SacI-EcoRI issu du plasmide pMC1396 (M. Casadaban et al. J. Bacteriol. (1980) 143 : 971-980). Le plasmide pDIA5501 modifié contient le gène codant pour la $\beta$-galactosidase tronquée de son extrémité C-terminale. Il contient également l'origine de réplication et le gène codant pour la résistance à l'ampicilline provenant de pBR322.

Le plasmide obtenu sous 1.a. est digéré par les enzymes de restriction EcoRI et SalI; le fragment d'ADN contenant le gène codant pour le Trp-hGRF-OH est purifié par électrophorèse sur gel. Ce fragment est ensuite incubé en présence de l'ADN-ligase du bactériophage T4, avec le grand fragment EcoRI-SalI issu du plasmide pDIA5501 modifié, préalablement digéré totalement par l'enzyme SalI et partiellement par l'enzyme EcoRI. Un nouveau plasmide, le plasmide 202,17, est obtenu.

Le plasmide 202,17 contient le fragment d'ADN représenté sur la figure 2. La figure 3 en donne une représentation schématique; il comprend les éléments ci-après :

-éléments apportés par le fragment EcoRI-SalI du plasmide pDIA5501 modifié :

-un fragment SalI-EcoRI issu du plasmide pBR322 portant :

l'origine de réplication de pBR322,

le gène codant pour la résistance à l'ampicilline;

-un fragment EcoRI-SacI portant :

le gène codant pour le répresseur thermosensible CI 857,

le promoteur PR du bactériophage λ,

la partie du gène codant pour la $\beta$-galactosidase comprise entre les nucléotides codant pour l'extrémité N-terminale de la protéine et le site de coupure de l'enzyme SacI.

-un fragment SacI-EcoRI issu du plasmide pMC1396;

-élément apporté par le fragment EcoRI-SalI du plasmide obtenu sous 1.a., à savoir le gène codant pour le Trp-GRF-OH.

Le plasmide 202,17 porte donc un gène codant pour une protéine hybride constituée par la $\beta$-galactosidase modifiée unie au Trp-GRF-OH et dont l'expression dépend du promoteur PR lui-même placé sous le contrôle du répresseur CI 857.

1.c. Production de la protéine hybride $\beta$-galactosidase modifiée-Trp-hGRF-OH

La souche contenant le plasmide 202,17 est cultivée dans 15 l de milieu L contenant 50 µg/ml d'ampicilline, à 30°C, jusqu'à l'obtention d'une densité correspondant à 10 000 000 bactéries/ml; la culture est alors poursuivie pendant 2 à 3 h à 42°C. La synthèse de la protéine hybride est mise en évidence par l'apparition d'une bande spécifique correspondant à la taille de la $\beta$-galactosidase lorsque les protéines cellulaires sont analysées par électrophorèse sur gel de dodécylsulfate de sodium (SDS)-polyacrylamide suivie d'une coloration au bleu de Coomassie.

1.d. Extraction et purification de la protéine hybride $\beta$-galactosidase modifiée-Trp-hGRF-OH

20 ml de la culture bactérienne sont centrifugés. Le culot est repris dans 3 ml de tampon phosphate 100 mM à pH 7,8. Les bactéries ainsi remises en suspension sont soumises à une sonication. Le lysat bactérien obtenu est centrifugé : un premier surnageant S1 et un culot C1 sont obtenus. Le culot C1 est repris dans 1 ml d'un détergent de type ester alkylarylique (Triton) à 0,1%; la suspension résultante est centrifugée; un second surnageant S2 et un culot C2 sont obtenus. Le culot C2 est repris dans 0,5 ml de SDS à 1%; la suspension résultante est centrifugée : un troisième surnageant S3 et un culot C3 sont obtenus. Le culot C3 est repris dans 0,5 ml de SDS à 1%; la suspension résultante est centrifugée : un quatrième surnageant S4 est obtenu.

Les surnageants S2, S3 et S4 sont regroupés. Leur pool (pool A) est soumis à un gel-filtration sur gel Sephadex G25 équilibré en acide acétique à 70%. Les fractions d'élution sont recueillies; une mesure de la densité optique (DO) à 280 nm en continu est effectuée. Les fractions éluant dans le volume mort de la colonne et correspondant au pic protéique sont regroupées; leur pool (pool B) contient la protéine hybride.

1.e. Coupure de la protéine hybride $\beta$-galactosidase modifiée-Trp-hGRF-OH par le BNPS-scatole

La concentration en protéines du pool B est déterminée par la méthode de Bradfort (Kit BIO-RAD Protein Assay). Au pool B est ajouté du BNPS-scatole (PIERCE Chemical, Co., Rockford, Il., USA) à raison de 200 mg de BNPS-scatole pour 100 mg de protéines totales; après 24 h de contact à température ambiante, à l'abri de la lumière et avec une agitation intermittente, le pool B est soumis à une extraction renouvelée ensuite cinq fois par l'éther diéthylique. La phase aqueuse finale résultant de cette extraction est chromatographiée sur une colonne PERKIN-ELMER/HS-5 C18. Deux tampons (tampon A : $H_2O$ 99,9% et acide trifluoroacétique (TFA) 0,1%; tampon B : acétonitrile 99,9% et TFA 0,1%) sont utilisés pour préparer un gradient linéaire (de 15% de tampon B à 80% de tampon B) obtenu en 15 min avec un débit de 2 ml/min.

Les fractions d'élution sont recueillies et une mesure de DO à 220 nm (spécifique de la liaison peptidique) est effectuée en continu.

La courbe d'élution des fractions obtenues est représentée sur la figure 4, sur laquelle on a porté en abscisses, le temps d'élution en minutes et, en ordonnées, la densité optique DO. La droite A représente le gradient d'élution défini ci-dessus.

1.f. Caractérisation de l'hGRF-OH

Les fractions d'élution, dont certaines sont regroupées, sont lyophilisées (pour éliminer le TFA)-,reprises par 50 µl d'eau distillée et soumises à un dosage radio-immunologique du type RIA (Radio-Immuno-Assay).

Ce test RIA se fonde sur la mise en compétition de l'hGRF-OH obtenu selon l'invention et de l'hGRF-OH obtenu par voie de synthèse chimique et marqué à l'125I, en présence d'une faible quantité d'anticorps préparés contre l'hGRF-(1-40).

Il a été constaté que l'hGRF-OH obtenu selon l'invention est contenu dans les fractions recueillies entre les temps d'élution 3 et 7 min 30 s, comme cela apparaît sur la figure 4 sur laquelle on a également indiqué la teneur en GRF-OH des fractions recueillies entre les temps d'élution 3 et 8 min, l'ordonnée de droite étant l'échelle correspondante (GRF-OH en µg/ml déterminé par RIA).

1.g. Activité biologique

Un test d'activité biologique a été réalisé sur l'une des fractions ci-dessus.

Dans ce test, trois rats ont reçu chacun, en injection intraveineuse, 0,5 µg d'hGRF-OH selon l'invention. Trois autres rats ont reçu chacun, par injection intraveineuse, 0,5 µg de hGRF obtenu par synthèse chimique. Aux temps to (moment de l'injection), to + 2 min, to + 5 min, to + 10 min, to + 15 min et to + 30 min, un dosage de l'hormone de croissance contenue dans le plasma (recueilli par prélèvement intracardiaque) de chaque rat a été réalisé. Les courbes d'activité obtenues à partir de ces mesures indiquent que l'hGRF-OH obtenu selon l'invention induit chez le rat une réponse similaire à celle qui est induite par l'hGRF.

**Revendications**

1 -Procédé d'obtention de dérivés non amidés de la somatocrinine de formule GRF(1-43)-X, dans laquelle X est la leucine, la glycine, la valine ou l'alanine par génie génétique, caractérisé en ce qu'il consiste à :

1) préparer le fragment d'ADN codant pour le Trp-GRF(1-43)-X, dans lequel X est tel que défini ci-dessus;

2) insérer ledit fragment dans un vecteur contenant le gène codant pour la protéine porteuse avec les éléments génétiques nécessaires à son expression, l'insertion étant réalisée au niveau de la séquence codant pour la partie carboxylique terminale de ladite protéine porteuse;

3) transformer une cellule hôte avec le vecteur recombinant résultant;

4) faire exprimer le polypeptide de formule I :

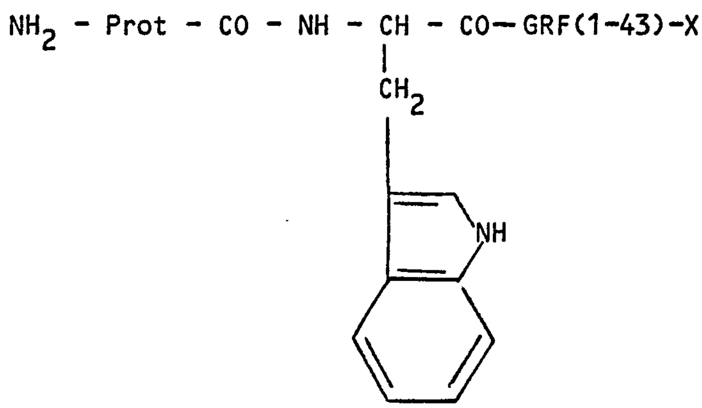

(I)

X = Leu, Ala, Val ou Gly;

Prot = résidu de la protéine porteuse;

5) récupérer ledit polypeptide de formule I et le couper au niveau du tryptophane à l'aide d'un agent oxydant pour obtenir le composé de formule GRF(1-43)-X, dans laquelle X est tel que défini ci-dessus.

2 -Procédé selon la revendication 1, caractérisé en ce que le fragment d'ADN codant pour le Trp-GRF(1-43)-X est obtenu par :

1) synthèse chimique d'un oligonucléotide portant le codon du tryptophane;

2) ligation dudit oligonucléotide au gène codant pour le GRF(1-43)-X porté par un vecteur, à l'extrémité correspondant à la partie N terminale de celui-ci.

3 -Procédé selon l'une des revendications 1 ou 2, caractérisé en ce que la protéine porteuse est la $\beta$-galactosidase, un analogue ou un fragment de ceux-ci et en ce que la cellule hôte est Escherichia coli.

4 -Vecteur recombinant codant pour la protéine hybride constituée par le GRF-OH lié par son extrémité N terminale à une protéine porteuse par l'intermédiaire du tryptophane, ladite protéine hybride répondant à la formule :

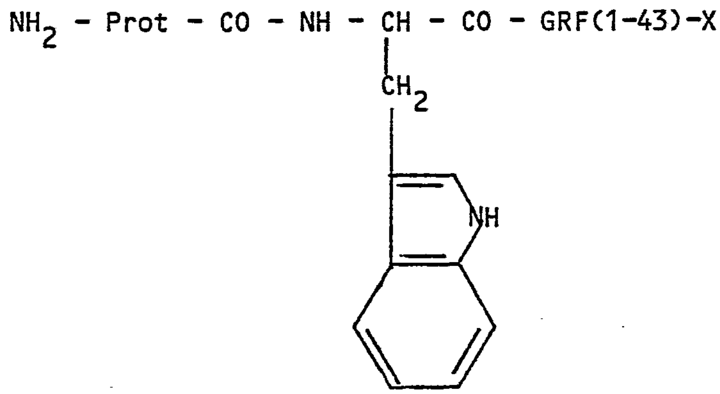

(I)

dans laquelle Prot et X sont tels que définis dans la revendication 1.

5 -Vecteur recombinant selon la revendication 4, caractérisé en ce que la protéine porteuse est la $\beta$-galactosidase.

6 -Vecteur recombinant selon la revendication 5, caractérisé en ce qu'il est le plasmide 202,17.

7 -Protéine hybride de formule :

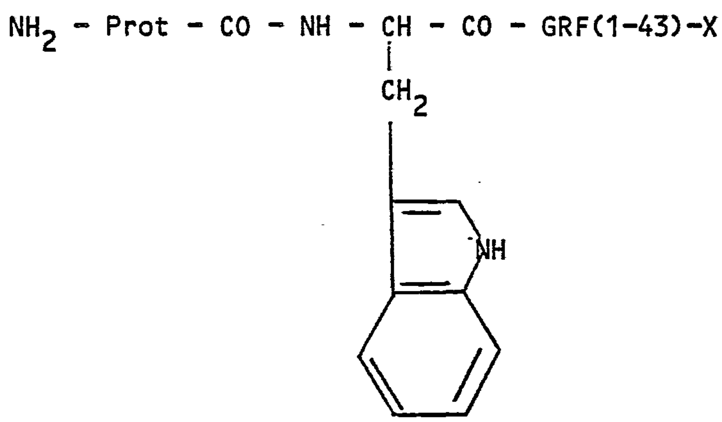

(I)

dans laquelle X est tel que défini dans la revendication 1, Prot est le résidu de la protéine porteuse.

8 -Protéine hybride selon la revendication 7, caractérisée en ce que Prot est un résidu de la $\beta$-galactosidase.

9 -Polypeptide de formule GRF(1-43)-X, dans laquelle X est la leucine, l'alanine, la valine ou la glycine.

10 -Souches d'Escherichia coli contenant le vecteur recombinant selon l'une quelconque des revendications 4 à 6.

11 -Souche selon la revendication 10, telle que déposée à la Collection Nationale de Cultures de Micro-organismes à Paris le 22 octobre 1984 sous le n° I-354.

Fig-1

```
RsaI
       1  2  3  4  5  6  7  8  9  10 11 12 13 14 15 16 17 18 19 20 21 22
    MetTyrAlaAspAlaIlePheThrAsnSerTyrArgLysValLeuGlyGlnLeuSerAlaArgLysLeu
AATTCATGTACGCAGACGCTATCTTTACTAACTCTTACCGTAAAGTTCTGGGCCAGCTGTCTGCACGCAAGCTT
TTAAGTACATGCGTCTGCGATAGAAATGATTGAGAATGGCATTTCAAGACCCGGTCGACAGACGTGCGTTCGAA

23 24 25 26 27 28 29 30 31 32 33 34 35 36 37 38 39 40 41 42 43 44
LeuGlnAspIleMetSerArgGlnGlnGlyGluSerAsnGlnGluArgGlyAlaArgAlaArgLeu Stop
CTGCAGGATATCATGTCTAGACAGCAGGGCGAATCTAACCAGGAGCGTGGCGCCCGTGCACGCCTGTAGGATCCGTCGAC
GACGTCCTATAGTACAGATCTGTCGTCCCGCTTAGATTGGTCCTCGCACCGCGGGCACGTGCGGACATCCTAGGCAGCTG
```

Fig. 2

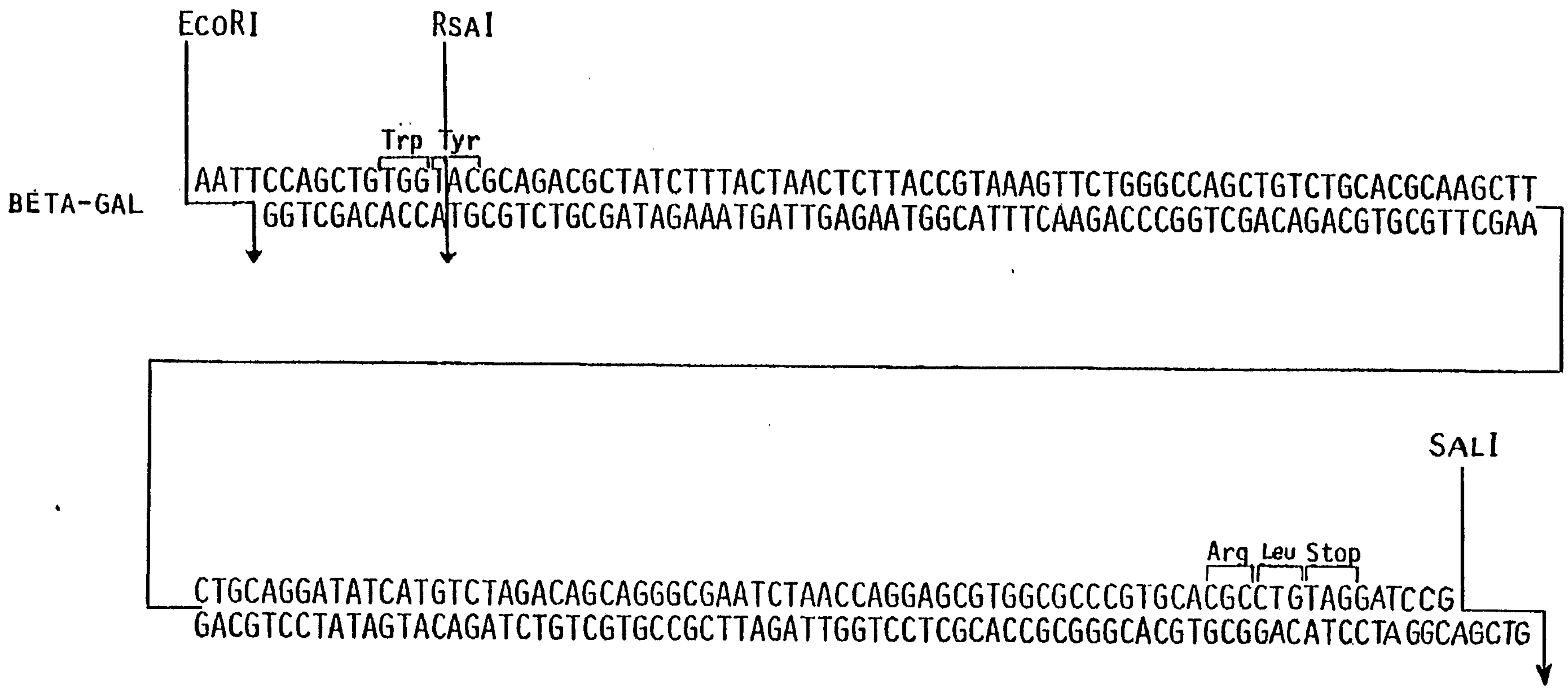
EcoRI
RsaI
Trp Tyr
BETA-GAL
AATTCCAGCTGTGGTACGCAGACGCTATCTTTACTAACTCTTACCGTAAAGTTCTGGGCCAGCTGTCTGCACGCAAGCTT
GGTCGACACCATGCGTCTGCGATAGAAATGATTGAGAATGGCATTTCAAGACCCGGTCGACAGACGTGCGTTCGAA
SALI
Arg Leu Stop
CTGCAGGATATCATGTCTAGACAGCAGGGCGAATCTAACCAGGAGCGTGGCGCCCGTGCACGCCTGTAGGATCCG
GACGTCCTATAGTACAGATCTGTCGTGCCGCTTAGATTGGTCCTCGCACCGCGGGCACGTGCGGACATCCTAGGCAGCTG

Fig-3

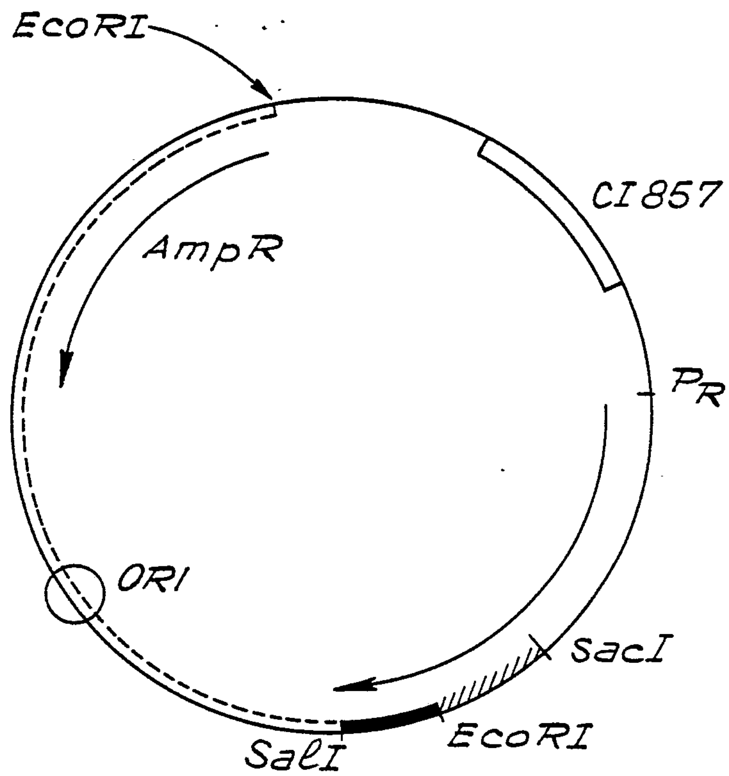


ORI : ORIGINE DE REPLICATION

====== GRAND FRAGMENT EcoRI-SalI de pBR 322

▬▬ GÈNE CODANT Trp-GRF(1-43)-LEU (Trp-GRF-OH)

—— FRAGMENT EcoRI-SacI du PLASMIDE pDIA 5501 AVEC LE PROMOTEUR $P_R$ ET LE GÈNE CI 857

////// FRAGMENT SacI-EcoRI DE pMC 1396

Fig-4

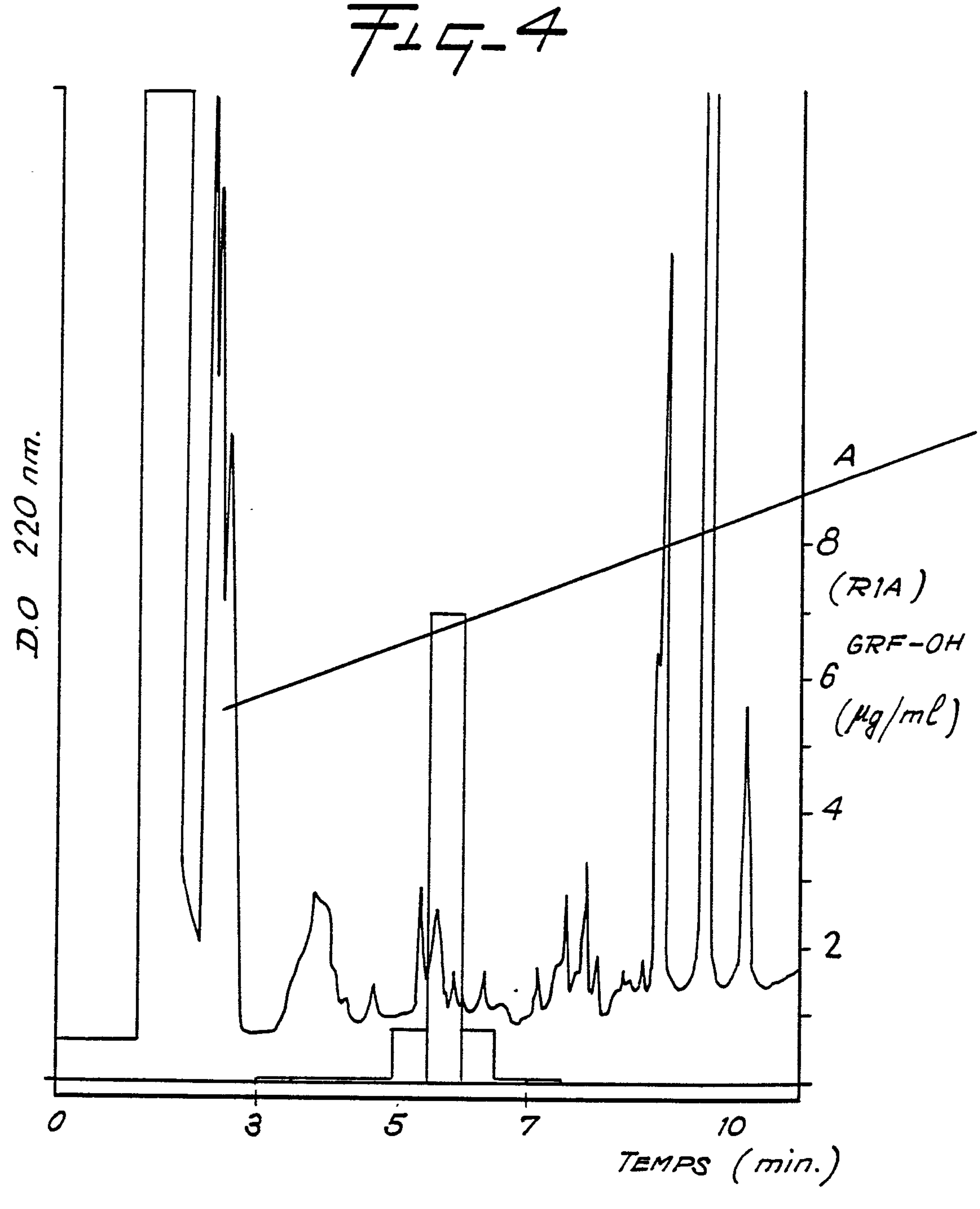

Office européen des brevets

# RAPPORT DE RECHERCHE EUROPEENNE

Numero de la demande

EP 86 40 1110

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | PROC. NATL. ACAD. SCI. USA, vol. 80, juillet 1983, pages 4311-4314; U. GUBLER et al.: "Cloning and sequence analysis of cDNA for the precursor of human growth hormone-releasing factor, somatocrinin" * En entier * --- | 1 | C 12 N 15/00<br>C 12 P 21/02<br>C 12 N 1/20 //<br>(C 12 N 1/20<br>C 12 R 1:19 ) |
| A | NATURE, vol. 306, no. 5938, 3 novembre 1983, pages 86-88, MacMillan Journals Ltd., Chesham, Bucks, GB; K.E. MAYO et al.: "Expression-cloning and sequence of a cDNA encoding human growth hormone-releasing factor" * En entier * --- | 1 | DOMAINES TECHNIQUES RECHERCHES (Int. Cl.4) |
| A | FEDERATION PROCEEDINGS, vol. 42, no. 7, 1983, résumé no. 1160; P.J. BARR et al.: "The chemical synthesis and expression of genes for human growth hormone releasing factor (somatocrinin)" * En entier * --- | 1 | C 12 N |
| A | EP-A-0 108 387 (HOFFMANN LA ROCHE) * Revendications * --- -/- | 1 | |

Le présent rapport de recherche a été établi pour toutes les revendications

| Lieu de la recherche | Date d'achèvement de la recherche | Examinateur |
|---|---|---|
| LA HAYE | 01-09-1986 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 86 40 1110



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 129 073 (CHIRON)<br>* Revendications *<br>--- | 1 | |
| A | BE-A- 898 666 (REGION WALLONNE)<br>* Revendications *<br>--- | 1 | |
| A | EP-A-0 133 282 (HOECHST)<br>* Revendications *<br>----- | 1 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 01-09-1986 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

Numéro de la demande :

86401110.1

## DECLARATION SELON LA REGLE 28, PARAGRAPHE 4 DE LA CONVENTION SUR LE BREVET EUROPEEN

Le demandeur a informé l'Office européen des brevets que, jusqu'à la publication de la mention de la délivrance du brevet européen ou jusqu'à la date à laquelle la demande est rejetée, retirée ou réputée retirée, l'accessibilité au(x) micro-organisme(s) identifié(s) ci-dessous, visée au paragraphe 3 de la règle 28 de la Convention sur le brevet européen, ne peut être réalisée que par la remise d'un échantillon à un expert.

## IDENTIFICATION DES MICRO-ORGANISMES

**Numéros d'ordre des dépôts :** I - 354